# EUROPÄISCHE PATENTSCHRIFT

(11) **EP 1 093 780 B1**
(45) Veröffentlichungstag und Bekanntmachung des Hinweises auf die Patenterteilung: **26.11.2003**
(21) Anmeldenummer: 00119627.8
(22) Anmeldetag: 08.09.2000
(51) Int. Cl.: A61F 13/02

(54) **Verbandmaterial auf Folienbasis mit Aufdruck**
Foil based dressing with print
Pansement à base d'un film avec imprimé

(30) Priorität: 19.10.1999 DE 19950295
(43) Veröffentlichungstag der Anmeldung: 25.04.2001
(73) Patentinhaber: Beiersdorf AG, 20253 Hamburg (DE)
(72) Erfinder: Trotter, Sebastian, 21244 Buchholz (DE); Bruss, Witta, 86199 Augsburg (DE)

(56) Entgegenhaltungen:
- EP-A- 0 424 165
- WO-A-88/08787
- DE-A- 4 314 834

## Beschreibung

Die Erfindung betrifft ein Verbandmaterial auf Folienbasis mit Aufdruck sowie Verfahren zur Herstellung des bedruckten Verbandmaterials.

Verbandmaterialien auf Folienbasis sind bekannt. Insbesondere bestehen die Folien aus Polyurethan. Diese PU-Folien werden u.a. als Trägermaterialien für Pflaster zur Abdeckung von Wunden eingesetzt.

Die Herstellung der PU-Folienpflaster erfolgt, indem eine PU-Dispersion (vorzugsweise Impranil ® der Fa. Bayer AG) auf einen geeigneten Hilfsträger, vorzugsweise eine oberflächengeprägte PE-Folie, flächig ausgestrichen und zur Filmbildung getrocknet wird. Das Ausstreichen und Trocknen kann mehrfach erfolgen, so daß ein schichtweiser Aufbau entsteht, wobei die Schichten visuell unerkennbar und untrennbar sind.

Der so erhaltene Verbund Hilfsträger/PU-Film(e) kann auf der dem Hilfsträger abgewandten Seite mit einer geeigneten Klebemasse beschichtet und mit Trennpapier abgedeckt, zur Mutterrolle aufgeschnitten und durch Auflage einer Wundabdeckung und Eindeckpapier zu Einzelpflastern endkonfektioniert werden.
Das auf die dargelegte Art erzeugte Pflaster ist je nach Schichtstärke und ausgewählter Klebemasse transparent bis opak, die mittig aufgebrachte Wundauflage zeichnet sich unter der Trägerfolie ab.

Bei der Applikation der Pflaster wird zur Ausnutzung der flexiblen Eigenschaften der PU-Folie der stabilisierende PE-Hilfsträger dekaschiert.

Solche Pflaster werden im Stand der Technik umfassend beschreiben.

Die DE 40 26 755 A1 offenbart ein Verbandmaterial auf Folienbasis, das auf der einen Seite mit einem Stützmaterial abgedeckt ist, das die gleiche Größe wie die Folie besitzt und mindestens eine Griffleiste aufweist, und auf der anderen Seite mit einer selbstklebenden Schicht versehen ist. Die Griffleisten zur Entfernung des Trägermaterials sind innerhalb seiner Umfangsbegrenzung angeordnet. Vorzugsweise ist nur eine Griffleiste auf dem Trägermaterial angebracht.
Die DE 43 14 834 C2 beschreibt ein Verbandmaterial auf Folienbasis, das auf der einen Seite mit einem Trägermaterial abgedeckt ist, das die gleiche Größe wie die Folie besitzt und mindestens eine Griffleiste aufweist, und auf der anderen Seite mit einer selbstklebenden Schicht versehen ist. Die Griffleisten zur Entfernung des Trägermaterials sind innerhalb seiner Umfangsbegrenzung angeordnet. Vorzugsweise ist nur eine Griffleiste auf dem Trägermaterial angebracht.

Es ist vorteilhaft, die Trägermaterialien von Pflastern zu bedrucken, zum Beispiel für Jugendliche und Kinder, die der Anwendung von Pflastern eher ablehnend gegenüberstehen. Denn mit Pflastern werden negative Erinnerungen wie Schmerz, eventuell Bluten oder Verletzungen verbunden.
Um den optischen Reiz für Kinder und Jugendliche zu erhöhen, können die Aufdrucke insbesondere mit mindestens einer Comikfigur versehen werden. Die auf dem Markt anzutreffenden bedruckten Folienpflaster bestehen hauptsächlich aus PVC, PE, PET und anderen oligomeren Trägerfolien, die meistens dicker, inflexibler, unanschmiegsamer, härter, wasserdampfundurchlässig im Vergleich zu den u.a. in der DE 40 26 755 A1 und der DE 43 14 834 C2 beschriebenen PU-Folien sind.

Die DE G 74 20 413 beschreibt eine Plakatplakette mit einer zwei- oder dreidimensionalen Wiedergabe von mindestens einer Kindern bekannten und vorzugsweise bei diesen beliebten figürlichen Darstellung an ihrer Sichtoberfläche, die dadurch gekennzeichnet ist, daß sie als Wundpflaster mit einer das Trägermaterial für die figürliche Darstellung bildenden und auf der Haut zum Haften bringbare Bereiche aufweisenden Deckschicht aus wundfreundlichem und/oder heilungsförderndem und/oder atmungsaktivem Material ausgebildet ist. Auch diese Plakatplakette weist regelmäßig geformte Träger auf, auf die die gewünschte figürliche Darstellung gedruckt wird.

Allgemein weisen Pflaster aber überwiegend keinen Aufdruck auf der körperabgewandten Seite des Trägermaterials des Pflasters auf.
Erst seit neuerer Zeit sind derartige Pflaster am Markt anzutreffen. So werden sogenannte Junior-Strips ® mit einem Aufdruck, der insbesondere aus Darstellungen von gezeichneten Figuren besteht, auf einem Pflaster der herkömmlichen Aufmachung vertrieben, das sich insbesondere bei Kindern größter Beliebtheit erfreut.

Die DE 197 09 606 A1 beschreibt des weiteren ein Pflaster zum Verkleben auf der Haut, insbesondere zur Abdeckung von kleineren Wunden, das aus einem Trägermaterial besteht, das auf der unteren Seite mit einer hautverträglichen selbstklebenden Schicht versehen ist. Auf der oberen Seite des Trägermaterials - vorzugsweise eine Polyethylenfolie - ist vollflächig oder partiell ein langnachleuchtender Aufdruck vorhanden, der von einem Lacksystem gebildet wird, in das ein langnachleuchtendes Pigment eingearbeitet ist.

Des weiteren wird offenbart ein Verfahren zur Herstellung eines derartigen Pflasters, bestehend aus den folgenden Verfahrensschritten:
a) zumindest einmaliges vollflächiges Bedrucken der oberen Seite des Trägermaterials mit weißer Flexodruckfarbe im Flexodruck oder Siebdruck,
b) gegebenenfalls Bedrucken der im Verfahrensschritt d) nicht zu bedruckenden Teilbereiche der oberen Seite des Trägermaterials im Flexodruckverfahren,
c) Herstellung der Mischung aus einem Lacksystem und dem nachleuchtenden Pigment,
d) Bedrucken der oberen Seite des Trägermaterials mit der Mischung vorzugsweise im Siebdruck,
   bei partiellem Bedrucken Füllung der im Verfahrensschritt b) ausgesparten weißen Bereiche über ein Siebwerk,
e) gegebenenfalls Nachvemetzen der Mischung mittels UV-Strahlung oder thermisch.

Aufgabe der Erfindung ist es, ein Verbandmaterial auf Folienbasis zur Verfügung zu stellen, das eine Bedruckung trägt, insbesondere eine solche, die im Konterdruckverfahren aufgebracht worden ist.

Gelöst wird diese Aufgabe durch ein Verbandmaterial auf Folienbasis, wie es im Hauptanspruch niedergelegt ist. Gegenstand der Unteransprüche sind dabei vorteilhafte Weiterbildungen des Verbandmaterials sowie Verfahren zur Herstellung des erfindungsgemäßen Verbandmaterials.

Demgemäß betrifft die Erfindung ein Verbandmaterial auf Folienbasis, das auf der wundabgewandten Seite mit einem Trägermaterial abgedeckt ist, das die gleiche Größe wie die insbesondere transparente Folie besitzt, wobei auf der wundzugewandten Seite der transparenten Folie ein Aufdruck vorhanden ist.
Der Aufdruck wird dabei insbesondere im Konterdruckverfahren aufgebracht. Beim Konterdruckverfahren wird ein spiegelverkehrter Druck eingesetzt. Die Betrachtung der Bedruckung/Beschriftung wird von der wundabgewandten Seite durch die Folie hindurch wird diese in normaler Form erkannt.

Durch die Bedruckung können zum Beispiel Muster aufgebracht werden, oder die Bedruckung kann vollflächig erfolgen, so daß die Farbe des Trägers an die Haut angepaßt und damit das Produkt unauffälliger gestaltet werden kann. Insbesondere sind hierbei die wirtschaftlichen und kostengünstigen Vorteile gegenüber eines Einfärbevorgangs jeglicher Arten von Trägermaterialien zu erwähnen. Des weiteren besteht eine hohen Flexibilität in der Auswahl der Farben bei mengenunabhängigen gleichbleibenden Kosten.

Ein weiterer Vorteil besteht in der optischen Kaschierung (durch Bedrucken an der entsprechenden Wundauflagenposition des Trägermaterials) der Wundauflage, die im Falle einer stärkeren Blutung der Wunde durchtränkt und von außen optisch als störend empfunden wird.

In einer ersten bevorzugten Ausführungsform des Verbandmaterials ist auf der transparenten Folie mit dem Aufdruck zumindest eine weitere Folie vorhanden, die somit den Aufdruck abdeckt.
Damit befindet sich die Druckfarbe geschützt zwischen den Folien und hat keinen direkten Kontakt zur gegebenenfalls nachfolgend aufgebrachten Klebemasse und deren Hilfsstoffe. Von Vorteil ist hierbei, daß der Einsatz jeglicher Druckfarbe oder Beschichtung, beispielsweise UV-Druckfarben, Thermodruckfarben, besondere Beschichtungen von Mikrokapsein etc. möglich wird.
Diese schützende Abdeckung der Bedruckung schließt Wechselwirkungen aus, die sich zwischen der bedruckten PU-Filmseite mit den lösemittelbasierten Klebemassen, den UV-Klebmassen, den Dispersionsklebmassen und/oder den thermoplastischen Klebemassen wie Hotmelts ergeben können. Auch Wechselwirkungen zwischen den Klebmassen sowie den gegebenenfalls vorhandenen Lösemitteln, Pigmenten, Bindemitteln oder Additiven in der Druckfarbe können nicht auftreten. Insbesondere bei jahrelanger Haltbarkeit, die im Falle von Pflastern erwartet wird, werden alle Beeinträchtigungen der Druckqualität oder der Klebeeigenschaften unterdrückt.

Weiterhin bevorzugt ist ein Verbandmaterial, bei dem die wundzugewandte Seite mit einer selbstklebenden Schicht versehen ist, auf der gegebenenfalls eine Wundauflage angeordnet ist.
Die Klebschicht auf der Folie weist beispielsweise bevorzugt eine Klebkraft auf Stahl von etwa 2 bis 4 N/cm auf, wobei das Prüfmaterial, da die Folie sehr dehnbar ist, für die Messung rückseitig mit einem unelastischen Klebefilm verstärkt werden muß. Die Messung selbst erfolgte in Anlehnung an DAB 9.

Auf seiner vorzugsweise selbstklebend ausgerüsteten, später der Haut zugewandten Seite ist das erfindungsgemäße Verbandmaterial über seine ganze Breite bis zum Gebrauch üblicherweise mit einem klebstoffabweisenden Trägermaterial, wie silikonisiertes Papier, abgedeckt. Dieses schützt die Selbstklebeschicht aus einer gut hautverträglichen Klebemasse, beispielsweise auf Acrylatbasis, die vorzugsweise im Transferverfahren aufgebracht worden ist, und stabilisiert zusätzlich das ganze Produkt. Die Abdeckung kann in bekannter Weise einstückig oder vorzugsweise zweiteilig ausgebildet sein.

Das Verbandmaterial kann als solches verwendet werden, es kann jedoch auch zusätzlich mittig in geeigneter Breite eine übliche, saugende Wundauflage, welche kleiner als die Klebefläche ist, aufgebracht sein, so daß es direkt als Wundverband eingesetzt werden kann. Ein derartiger Verband mit Rundum-Verklebung ist besonders vorteilhaft, da er keimdicht und wasserfest ist.

Die Folie selbst besteht vorzugsweise aus elastischen, thermoplastischen Polyurethanen, wie sie in der DE 19 34 710 C beschrieben sind und die sich durch eine gute Hautverträglichkeit sowie Sauerstoff- und Wasserdampfdurchlässigkeit auszeichnen. Besonders vorteilhaft haben sich aliphatische Polyesterurethane erwiesen.

Eine bevorzugte Folie ist 30 bis 40 µm stark, transparent, weist eine Reißdehnung von über 450% und eine Wasserdampfdurchlässigkeit von über 500 g/m² in 24h bei 38 °C und 95% relativer Feuchte nach DAB auf.

Daneben lassen sich jedoch auch Filme auf anderer Grundlage, wie zum Beispiel Acrylat-Copolymere oder die anderen bekannten filmbildenden elastischen Polymeren, verwenden. Die Dicke der Folien kann dabei zwischen 15 bis 300 µm, vorzugsweise 15 bis 80 µm, das Gewicht entsprechend zwischen 15 bis 350 g/m², vorzugsweise 15 bis 100 g/m², die Höchstzugkraft längs zwischen etwa 5 bis 100 N/cm, vorzugsweise 2 bis 40 N/cm, und die Reißdehnung längs zwischen 100 bis 1000% betragen.

Das Trägermaterial, das eine stützende Wirkung für die Folie hat, beim Aufbringen des Verbandes auf dieser verbleibt und erst nachträglich entfernt wird, besteht vorzugsweise aus einer Polyethylenfolie von etwa 80 µm Dicke, die auf ihrer Seite zur Folie hin leicht gerauht ist und dadurch matt, aber noch durchscheinend wirkt.
Es lassen sich auch andere Folien aus beispielsweise Polypropylen, Polyester, PVC oder geeignetes dünnes, beschichtetes Papier verwenden, sofern sie nur schmiegsam genug sind, um beim Anbringen des Verbandes nicht zu stören.

Die Haftung der Folie auf dem Trägermaterial sollte zwischen 0,01 und 0,5 N/cm betragen_und damit wesentlich geringer sein als die Haftung der Klebeschicht auf der Haut nach Applikation des Verbandmaterials.

Die technischen Daten der Folie können sich in folgenden Bereichen bewegen:

| | |
|---|---|
| Dicke | 30 bis 300 µm |
| Gewicht | 30 bis 350 g/m² |
| Höchstzugkraft längs | 5 bis 100 N/cm |
| Reißdehnung längs | 10 bis 1000% |

Ihre Oberfläche zur Folie kann glatt, gerauht oder leicht geprägt sein.

In einer weiteren bevorzugten Ausführungsform des Verbandmaterials ist auf dem Trägermaterial zumindest eine Griffleiste vorhanden.

Die Griffleisten können dabei innerhalb der Umfangsbegrenzung des Trägermaterials angeordnet sein. D.h., die vorzugsweise nur eine Griffleiste, die auf dem Trägermaterial an beliebiger Stelle angebracht ist, steht nicht über dessen Rand hinaus und geht damit nicht verloren, wenn aus dem vorgefertigten Material kleinere Pflaster in allen möglichen Formen ausgestanzt werden. Vorzugsweise erstreckt sich die Griffleiste über die ganze Länge oder Breite des Pflasters, je nach dessen Form.
Vorteilhafterweise ist sie in der Weise auf dem Trägermaterial befestigt, daß sie in einem schmalen Randbereich angeklebt ist und einseitig links oder rechts davon einen freien, nichtverklebten Anfaßbereich von mindestens etwa 5 mm aufweist.
Die Griffleiste kann das ganze Pflaster bedecken, insbesondere wenn dieses sehr klein ist, sie kann es aber auch nur teilweise bedecken.
Bei einer der möglichen und bevorzugten Ausführungsformen schließt sie dabei bündig an einem Seitenrand ab.
Eine andere Ausführungsform ist in der Weise ausgebildet, daß die Griffleiste in einem mittigen schmalen, streifenförmigen Bereich auf dem Trägermaterial befestigt ist und beidseitig davon einen freien Anfaßbereich von mindestens etwa 5 mm Breite aufweist.

Des weiteren können separate Griffleisten entlang zweier gegenüberliegender Ränder des Trägermaterials fest angebracht sein, und zwar derart, daß die Griffleisten nur mit einem Teil ihrer Breite mit dem Trägermaterial verbunden sind und mit dem anderen, vorzugsweise größeren Teil ihrer Breite, über das Verbandmaterial hinausragen.

Die Befestigung der Griffleisten an dem Trägermaterial kann je nach Material und Verarbeitungsmaschinen in verschiedenster Weise erfolgen, vorzugsweise durch Kleben oder Verschweißen. Das Kleben erfolgt dabei beispielsweise in der Weise, daß zwischen das Stützmaterial und den Griffleistenstreifen im zu verklebenden Bereich ein Streifen eines doppelseitig klebenden Klebebandes eingebracht wird oder eine Klebemassenbeschichtung aus einer Hotmelt-Masse oder einer Selbstklebemasse aus Lösungsmittel oder Dispersion.

Dann umfaßt der Erfindungsgedanke auch Verfahren zur Herstellung eines erfindungsgemäßen Verbandmaterials, wobei
- die Folie zumindest in einer Schicht auf dem Trägermaterial erzeugt wird durch Gießen, Rakeln oder Extrudieren,
- die Folie auf der dem Trägermaterial abgewandten Seite bedruckt wird,
- auf den Druck der Folie gegebenenfalls eine weitere Folie durch Gießen, Rakeln oder Extrudieren aufgetragen wird,
- auf die Folie oder auf die weitere Folie eine selbstklebende Beschichtung aufgetragen wird.

Das erfindungsgemäße Verbandmaterial wird dann in der Regel einzeln in eine passend geformte Umhüllung eingesiegelt und gegebenenfalls durch Gamma-Strahlen sterilisiert.

Beim Gebrauch wird das Pflaster aus der Umhüllung entnommen, die Schutzabdeckung über der Klebschicht entfernt, das Pflaster auf die Wunde geklebt und anschließend das leicht wiederablösbare Trägermaterial auf der Rückseite der Folie mit der Griffleiste rückstandsfrei abgezogen.

Zur Handhabung des Verbandes bedarf es keiner Gebrauchsanleitung, da sich diese wie von selbst ergibt. Da der ganze Verband einschließlich der stützenden Trägerfolie zudem transparent ist, kann er ohne Schwierigkeiten paßgenau angebracht werden.

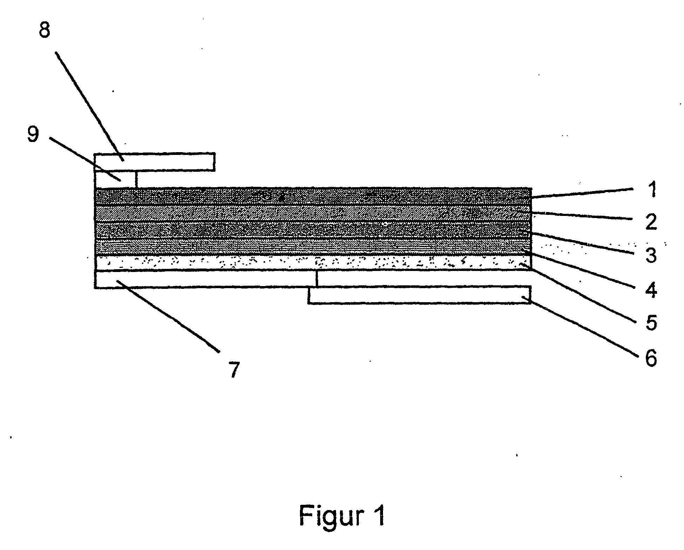

In der Figur 1 ist das erfindungsgemäße Verbandmaterial mit vergrößerten Dicken der Schichten beispielsweise dargestellt Dabei bedeuten
- (1): die Trägerschicht, vorzugsweise aus Polyethylen,
- (2): die erste Folie, vorzugsweise aus Polyurethan, wobei diese Schicht selbst aus mehreren einzelnen Lagen bestehen kann,
- (3): die Lage der Bedruckung,
- (4): die zweite Folie, vorzugsweise aus Polyurethan, wobei diese Schicht ebenfalls selbst aus mehreren einzelnen Lagen bestehen kann,
- (5): die Selbstklebeschicht,
- (6) und (7): die Schutzabdeckungen für die Selbstklebeschicht,
- (8): die an der Folie angeformte Griffleiste und
- (9): die Klebeschicht zur Befestigung der Griffleiste am Träger.

Im folgenden sollen anhand mehrerer Beispiele besonders vorteilhafte Ausführungsformen des Verbandmaterials dargestellt werden, ohne damit die Erfindung unnötig einschränken zu wollen.

### Beispiel 1

Das erfindungsgemäße Verbandmaterial besteht aus einer anionischen aliphatischen Polyesterpolyurethan-Dispersion-Folie (Impranil ® DLN Dispersion der Firma Bayer), und zwar in der Abmessung 50 x 50 mm. Die Folie weist eine Dicke von 0,1 mm auf.

Die Folie ist einseitig mit einer hautverträglichen Klebstoffschicht auf Basis vemetzter Polyacrylsäure-Derivate beschichtet.

### Beispiel 2

Das erfindungsgemäße Verbandmaterial wird analog zu Beispiel 1 von einem Polyurethangel "Cutinova thin" ® der Firma Beiersdorf AG gebildet, wobei dieser zusätzlich mit der Polyesterpolyurethan-Dispersion-Folie (Impranil ® DLN Dispersion der Firma Bayer) aus Beispiel 2 eingedeckt ist.

## Patentansprüche

1. Verbandmaterial auf Folienbasis, das auf der wundabgewandten Seite mit einem Trägermaterial (1) abgedeckt ist, das die gleiche Größe wie die Folie (2) besitzt, **dadurch gekennzeichnet, daß** auf der wundzugewandten Seite der Folie (2) ein Aufdruck (3) vorhanden ist.

2. Verbandmaterial gemäß Anspruch 1, **dadurch gekennzeichnet, daß** die Folie (2) mit dem Aufdruck (3) durch zumindest eine weitere Folie (4) abgedeckt ist.

3. Verbandmaterial gemäß den Ansprüchen 1 und 2, **dadurch gekennzeichnet, daß** die wundzugewandte Seite des Verbandmaterials mit einer selbstklebenden Schicht (5) versehen ist, auf der gegebenenfalls eine Wundauflage angeordnet ist, welche kleiner als die Klebefläche ist.

4. Verbandmaterial gemäß den Ansprüchen 1 bis 3, **dadurch gekennzeichnet, daß** die Folie (2) aus Polyurethan besteht.

5. Verbandmaterials gemäß zumindest einem der vorhergehenden Ansprüche, **dadurch gekennzeichnet, daß** auf dem Trägermaterial zumindest eine Griffleiste (8) vorhanden ist

6. Verfahren zur Herstellung eines Verbandmaterials gemäß zumindest einem der vorhergehenden Ansprüche, **dadurch gekennzeichnet, daß**
a) die Folie (2) zumindest in einer Schicht auf dem Trägermaterial (1) erzeugt wird durch Gießen, Rakeln oder Extrudieren,
b) die Folie (2) auf der dem Trägermaterial abgewandten Seite bedruckt wird,
c) auf den Druck der Folie gegebenenfalls eine weitere Folie (4) durch Gießen, Rakeln oder Extrudieren aufgetragen wird,
d) auf die Folie oder auf die weitere Folie eine selbstklebende Beschichtung (5) aufgetragen wird.

## Claims

1. Film-based dressing material lined on the side remote from the wound with a backing material (1) of the same size as the film (2), **characterized in that** the wound-facing side of the film (2) bears an imprint (3).

2. Dressing material according to Claim 1, **characterized in that** the film (2) bearing the imprint (3) is covered by at least one further film (4).

3. Dressing material according to Claims 1 and 2, **characterized in that** the wound-facing side of the dressing material is provided with a self-adhesive layer (5) on which, if desired, a wound pad smaller than the adhesive area is disposed.

4. Dressing material according to Claims 1 to 3, **characterized in that** the film (2) comprises polyurethane.

5. Dressing material according to at least one of the preceding claims, **characterized in that** there is at least one grip strip (8) on the backing material.

6. Process for producing a dressing material according to at least one of the preceding claims, **characterized in that**
a) the film (2) is produced in at least one layer on the backing material (1) by flow coating, knife coating or extrusion,
b) the film (2) is printed on the side facing away from the backing material,
c) if desired, a further film (4) is applied to the print of the film by flow coating, knife coating or extrusion,
d) a self-adhesive coating (5) is applied to the film or to the further film.

## Revendications

1. Matériau pour pansement à base d'un film, qui est revêtu sur le côté opposé à la plaie d'un matériau support (1), qui présente la même taille que le film (2), **caractérisé en ce qu'**une impression (3) se trouve sur le côté orienté vers la plaie du film (2).

2. Matériau pour pansement selon la revendication 1, **caractérisé en ce que** le film (2) avec l'impression (3) est recouvert par au moins un autre film (4).

3. Matériau pour pansement selon les revendications 1 et 2, **caractérisé en ce que** le côté orienté vers la plaie du matériau pour pansement est pourvu d'une couche auto-adhésive. (5), sur laquelle est le cas échéant disposé un enduit pour plaie, qui est plus petit que la surface adhésive.

4. Matériau pour pansement selon les revendications 1 à 3, **caractérisé en ce que** le film (2) est en polyuréthanne.

5. Matériau pour pansement selon au moins l'une quelconque des revendications précédentes, **caractérisé en ce qu'**au moins une languette de prise (8) se trouve sur le matériau support.

6. Procédé pour la production d'un matériau pour pansement selon au moins l'une quelconque des revendications précédentes, **caractérisé en ce que**
a) le film (2) est obtenu en au moins une couche sur le matériau support (1) par coulage, raclage ou extrusion,
b) le film (2) est imprimé sur le côté opposé au matériau support,
c) le cas échéant, un autre film (4) est appliqué sur l'impression du film par coulage, raclage ou extrusion,
d) un revêtement auto-adhésif (5) est appliqué sur le film ou l'autre film.
